# EUROPEAN PATENT APPLICATION

(11) **EP 3 632 207 A1**
(43) Date of publication of application: **08.04.2020**
(21) Application number: 18805534.7
(22) Date of filing: 24.05.2018
(51) Int. Cl.: A01N 1/02

(54) **CELL CRYOPRESERVATION FORMULATION AND CELL RECOVERY METHOD**

(30) Priority: 24.05.2017 CN 201710375424
(71) Applicant: Cellular Biomedicine Group (Shanghai) Ltd., Xuhui District, Shanghai 200233 (CN); Cellular Biomedicine Group (Wuxi) Ltd., Wuxi, Jiangsu 214174 (CN)
(72) Inventor: ZHANG, Li, Shanghai 200233 (CN); WANG, Fei, Shanghai 200233 (CN); HE, Jiaping, Shanghai 200233 (CN); LIN, Nanjing, Shanghai 200233 (CN); LIU, Liping, Shanghai 200233 (CN); LIU, Xin, Shanghai 200233 (CN); LI, Chao, Shanghai 200233 (CN); YU, Shuqian, Shanghai 200233 (CN); HU, Yonglai, Shanghai 200233 (CN); ZHAO, Jiawei, Shanghai 200233 (CN); SUN, Zhe, Shanghai 200233 (CN); LIU, Xiaoyu, Shanghai 200233 (CN)
(74) Representative: Mewburn Ellis LLP
(86) International application number: PCT/CN2018/088246
(87) International publication number: WO 2018/214943

(57) **Abstract**

The present invention relates to the preparation and use of a cell cryopreservation formulation convenient for clinical use. In particular, provided is a cell cryopreservation (freezing) formulation, comprising: (1) a cell; (2) a cryopreservation diluent; the cryopreservation diluent comprising: an aqueous solution of sodium chloride, protective proteins, and dimethyl sulfoxide. The invention also relates to a corresponding cell resuscitation method. The invention adopts a no-wash cryopreservation solution for preparation of a cell frozen preparation, and the cell resuscitation and dilution for clinical use can be carried out by a simple formulating operation.

## Description

### FIELD OF THE INVENTION

The present invention belongs to the field of cell therapy, and in particular, the present invention provides a no-wash frozen cell preparation for intravenous guttae, and simplified use of the frozen cell preparation.

### BACKGROUND OF THE INVENTION

When used in cell therapy, fresh cells are typically used for intravenous or topical infusion, or cryopreserved cells are used after wash. Fresh cells usually have an expiration date of less than 8 hours before clinical use, which is not conducive to long-distance transportation, and cannot be adapted to changes in clinical patients. Therefore, cryopreservation and resuscitation of cells are necessarily involved when cells are used in therapeutic field.

During cryopreservation and resuscitation in a laboratory, frozen cells are usually resuscitated and washed in a clean environment (such as in a sterile room) and a professional biotech technician is required for the operation. However, during the actual treatment, cryopreservation and resuscitation are carried out in a ordinary hospital, which is impossible to provide a sterile room environment, and lack of personnel with corresponding experimental skills, thus limiting the promotion of cell therapy.

In summary, there is an urgent need in the art for a simple cell cryopreservation and resuscitation method and corresponding reagents.

### SUMMARY OF THE INVENTION

In the invention, a no-wash cryopreservation solution is used for preparing a cell frozen preparation, and cells resuscitated and diluted by a simple formulating operation can be directly clinically used, thereby effectivly solving the above problems in the field of cell therapy.

In the first aspect of the invention, a cryopreserved (frozen) cell preparation is provided, which comprising:
(1) cells;
(2) A cryopreservation solution comprising: an aqueous solution of sodium chloride, a protective protein, and dimethyl sulfoxide.

In another preferred embodiment, the cells are selected from the group consisting of primary T cells, amplificated T cells, gene-transducted T cells, stem cells, or combinations thereof.

In another preferred embodiment, the cells are selected from the group consisting of suspension-cultured cells and momolayer-cultured cells.

In another preferred embodiment, the monolayer-cultured cells are pre-digested and suspended prior to preparation of the cryopreserved preparation.

In another preferred embodiment, the cell density in the formulation ranges from 1x10⁵-5x10⁸ / ml.

In another preferred embodiment, the concentration of sodium chloride in the preparation is from 0.85% to 0.95% (w/v).

In another preferred embodiment, the concentration of protective protein in the preparation is from 10% to 90% (w/v).

In another preferred embodiment, the concentration of protective protein in the preparation is from 10% to 30% (w/v).

In another preferred embodiment, the concentration of protective protein in the preparation is from 15% to 25% (w/v).

In another preferred embodiment, the concentration of dimethyl sulfoxide in the preparation is from 5% to 40% (w/v).

In another preferred embodiment, the concentration of dimethyl sulfoxide in the preparation is from 5% to 20% (w/v).

In another preferred embodiment, the concentration of dimethyl sulfoxide in the preparation is from 8% to 12% (w/v).

In another preferred embodiment, the method comprises the following steps:
(a) providing cells to be cryopreserved and a cryopreservation diluent, and resuspending the cells with the cryopreservation diluent to obtain a first cryopreservation mixture; wherein the cryopreservation diluent comprises following components: an aqueous solution of sodium chloride, and a protective protein;
(b) adding the cryopreservation solution to the first cryopreservation mixture, and mixing to obtain a second cryopreservation mixture;
(c) transferring the second cryopreservation mixture to a container and conducting a programmed cooling to below -80 °C to obtain the cryopreserved cell preparation.

In another preferred embodiment, the ratio of the volume of the cryopreservation solution described in the step (b) (Vb) to the volume of the cryopreservation diluent described in the step (a) (Va) is Vb: Va = 1: 0.8-1.2.

In another preferred embodiment, the container is a frozen bag or a frozen tube.

In another preferred embodiment, the cryopreservation diluent comprises: an aqueous solution of sodium chloride, and protective proteins.

In another preferred embodiment, the concentration of sodium chloride in the cryopreservation diluent is from 0.85% to 0.95% (w/v).

In another preferred embodiment, the concentration of the protective protein in the cryopreservation diluent is from 10% to 90% (w/v).

In another preferred embodiment, the concentration of protective protein in the preparation is from 10% to 30% (w/v).

In another preferred embodiment, the concentration of protective protein in the preparation is from 15% to 25% (w/v).

In a second aspect of the invention, a resuscitation method for cryopreserved cells is provided, comprising the steps:
(i) providing a cryopreserved cell preparation as described in the first aspect of the invention;
(ii) placing the cryopreserved cell preparation into a 35-40 °C constant temperature apparatus for resuscitation to obtain a resuscitated cell-cryopreservation solution complex;
(iii) transferring the cells in the preparation to an injection carrier with a syringe or a disposable connector.

In another preferred embodiment, the resuscitation further comprises the steps: detecting the viability of the cells, and clinically using the cells if the viability meets the requirements for use.

In another preferred embodiment, the constant temperature apparatus is a thermostatic water bath.

In another preferred embodiment, the thawing time for cells is from 30 seconds to 3 minutes.

In another preferred embodiment, the injection carrier is an intravenously injectable solution, preferably selected from the group consisting of sodium chloride injection, multiple electrolytes injection, and amino acid injection.

It should be understood that, in the present invention, each of the technical features specifically described above and below (such as those in the Examples) can be combined with each other, thereby constituting new or preferred technical solutions which need not be specified again herein .

### DESCRIPTION OF THE DRAWINGS

Figure 1 is a graph showing changes in cell viability pre-cryopreservation and post-resuscitation in example 1;
Figure 2 is a graph showing the cell proliferation ability after cryopreservation and resuscitation in example 1;
Figure 3 is a graph showing the comparison of tumor antigen response ability of CAR-T cells before and after cryopreservation in example 1;
Figure 4 is a comparison of different cryopreservation methods (programs).

### EMBODIMENTS FOR CARRYING OUT THE INVENTION

After long-term and in-depth research, the inventors designed a simple cell cryopreservation-resuscitation method and the corresponding cryopreservation solution used. The method can be conveniently performed by a medical professional in an ordinary laboratory environment, and thus can be used as a cryopreservation-resuscitation method in a cell therapy process. The present invention is completed based on the above findings.

### CRYOPRESERVATION CELL PREPARATION

As used herein, the terms "cryopreservation cell preparation" and "frozen cell preparation" can be used interchangeably, which refers to a preparation containing therapeutically active cells that is preserved at low temperatures (eg, at -80 °C, or in a liquid nitrogen environment).

The cryopreserved (frozen) cell preparation of the present invention comprises the following components:
(1) cells;
(2) A cryopreservation solution comprising: an aqueous solution of sodium chloride, a protective protein, and dimethyl sulfoxide.

The cells are active cells which can be used for cell therapy, and the type thereof is not particularly limited. In a preferred embodiment of the present invention, the cells are cells selected from the group consisting of primary T cells, amplificated T cells, gene transducted T cells, stem cells, or combinations thereof.

The cell type of the present invention is not particularly limited, and in another preferred embodiment, the cell is a cell selected from the group consisting of suspension-cultured cells and monolayer-cultured cells. Preferably, the monolayer-cultured cells are pre-digested and suspended prior to preparation of the cryopreserved preparation.

In the preparation, the cell density range is not particularly limited and may be, for example, 1×10⁵-5×10⁸/ml.

Preferably, the concentration of sodium chloride in the preparation is from 0.85% to 0.95% (w/v).

In another preferred embodiment, the concentration of protective protein in the preparation is from 10% to 90% (w/v).

In another preferred embodiment, the concentration of dimethyl sulfoxide in the preparation is from 5% to 40% (w/v).

### Cell cryopreservation and resuscitation method

The invention also provides a method for cryopreservation-resuscitation of cells, which comprises the steps:
(a) providing cells to be cryopreserved and the cryopreservation diluent, and resuspending the cells with the cryopreservation diluent to obtain a first cryopreservation mixture; wherein the cryopreservation diluent comprises the following components: an aqueous solution of sodium chloride, and a protective protein;
(b) adding cryopreservation solution to the first cryopreservation mixture, and mixing to obtain a second cryopreservation mixture;
(c) transferring the second frozen mixture to a container and conducting a programmed cooling to below -80 °C to obtain a frozen cell preparation.
wherein the ratio of the volume of the cryopreservation solution described in the step (b) (Vb) to the volume of the cryopreservation diluent described in the step (a) (Va) is Vb: Va = 1: 0.8-1.2. Of course, the ratio of the above two may be slightly different depending on the type of cells to be frozen, or conditions such as the freezing temperature.

The container is not particularly limited, and is preferably a container, the surface of which can be pierced with a syringe to perform liquid absorption. In a preferred embodiment of the invention, the container is a frozen bag or a frozen tube. With the above device, the pollution caused by the ordinary laboratory environment can be avoided to the utmost extent.

The cryopreserved cells can be resuscitated in a conventional non-sterile environment. In a preferred embodiment of the invention, the resuscitation process comprises the steps:
(i) providing the cryopreserved cell preparation;
(ii) placing the cryopreserved cell preparation to a 35-40 °C constant temperature apparatus for resuscitation to obtain a resuscitated cell-cryopreservation solution complex;
(iii) transferring the cells in the preparation to an injection carrier with a syringe or a disposable connector.

In another preferred embodiment, the resuscitation further comprises the steps: detecting the viability of the cells, and clinically using the cells if the viability meets the requirements for use.

The constant temperature apparatus is not particularly limited and may be a thermostatic device commonly used in a laboratory (such as a constant temperature water bath).

In another preferred embodiment, the thawing time for cells is from 30 seconds to 3 minutes.

The injection carrier is not particularly limited, and may preferably be an intravenously injectable solution, more preferably selected from the group consisting of sodium chloride injection, multiple electrolytes injection, and amino acid injection.

In a preferred embodiment of the invention, the collected cells are washed with physiological saline prior to cryopreservation to ensure that the residues meet the requirements on clinical use of the product. The cryopreservation diluent has the same composition as the cryopreservation solution except that it does not contain dimethyl sulfoxide. According to the cryopreservation specification, cells are resuspended with the frozen diluent to obtain a cell concentration 2 times of the final concentration, and then an equal volume of the cryopreservation solution is slowly added, and then well mixed. The cell suspension was transferred to a cryopreservation bag (or a cryopreservation tube), programmed cooling was conducted at a cooling rate of 1-2 °C/min, and after cooled to below -80 °C, transferred to a liquid nitrogen refrigerator for storage.

At the time for use, the frozen preparation was taken out from the liquid nitrogen refrigerator and rapidly thawed at 37 °C. After thawing, the cell preparation was transferred to physiological saline with an injection or a single use connector. The viability assay was performed on the diluted cell preparation. And the cells are used clinically where the viability meets the requirements for use.

### Compared with the prior art, main advantages of the present invention includes:

(1) A high cell viability can be maintained for the cell preparation prepared by the present invention after a long-term storage, which is convenient for long-distance transportation from a preparation unit to a hospital.
(2) All of the reagents used in the cell cryopreservation preparations according to the present invention are adjuvant-grade or pharmaceutical-grade reagents, which can be safely used in clinical applications.
(3) Strict requirements of the cell preparation operation on aseptic environment can be eliminated through resuscitation of the cryopreserved cell preparation by using the method of the present invention, thereby enabling an untrained nurse to easily grasp the resuscitation of the frozen cell and maintain high cell viability.
(4) Biological activitiesy of the cells can be maintained through the resuscitation operation of the cryopreserved cell preparation by using method of the present invention.

The present invention will be further illustrated below with reference to the specific examples. It should be understood that these examples are only to illustrate the invention but not to limit the scope of the invention. The experimental methods with no specific conditions described in the following examples are generally performed under the conventional conditions, or according to the manufacturer's instructions. Unless indicated otherwise, parts and percentage are calculated by weight.

### Example 1

The cryopreservation storage container of the cryopreservation cell formulation is a frozen storage bag. The cells are CAR-T cells. Cryopreservation specification is 2×10⁷/10 ml. The components of the cryopreservation cell preparation are shown in the table below. The CAR-T cells were cryopreserved for 3 months with 100 ml of preparation, and then thawed in a 37 °C water bath for 2 minutes, and the thawed cells were diluted with a syringe into 100 ml of physiological saline. The diluted cells were taken for viability assay, tumor antigen response assay and cell proliferation assay.

| No. | Component | content |
|---|---|---|
| 1 | cells | 2×10⁷/ml |
| 2 | Sodium chloride | 0.9% (w/v) |
| 3 | Human serum albumin | 20% (w/v) |
| 4 | Dimethyl sulfoxide | 10% (v/v) |

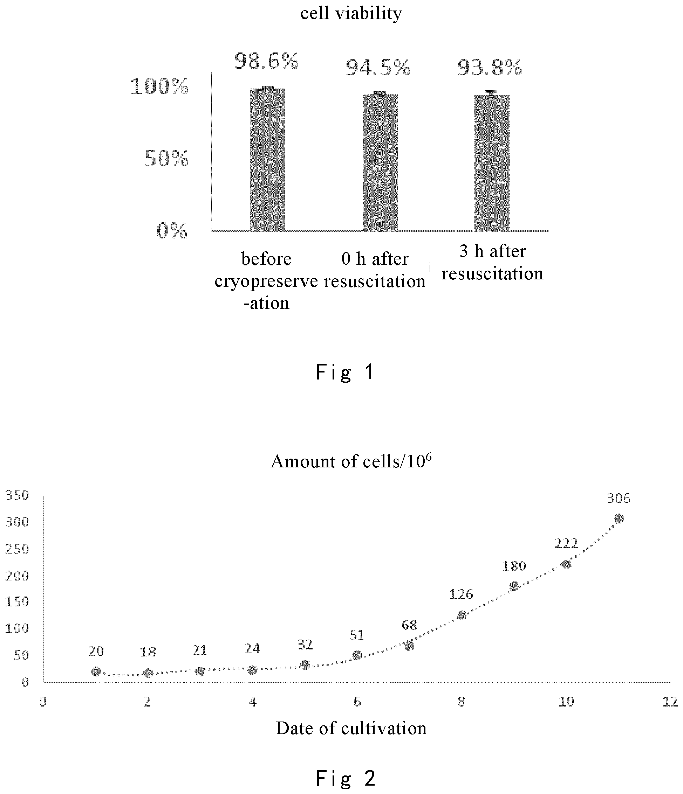

Changes in Cell viability: the diluted cell suspension was tested for viability by trypan blue staining and the results are shown in Figure 1. The results showed that the cells maintained a high survival rate after cryopreservation and resuscitation.

**Proliferation ability of cells after cryopreservation:** The cryopreserved and resuscitated cells were placed in complete medium and cultured, and the density of the cells was recorded daily. The medium was supplemented every other 2-3 days and a growth curve was drawn (as shown in Figure 2). It can be seen from the figure that after the cryopreservation and resuscitation, the cells still have better proliferative capacity.

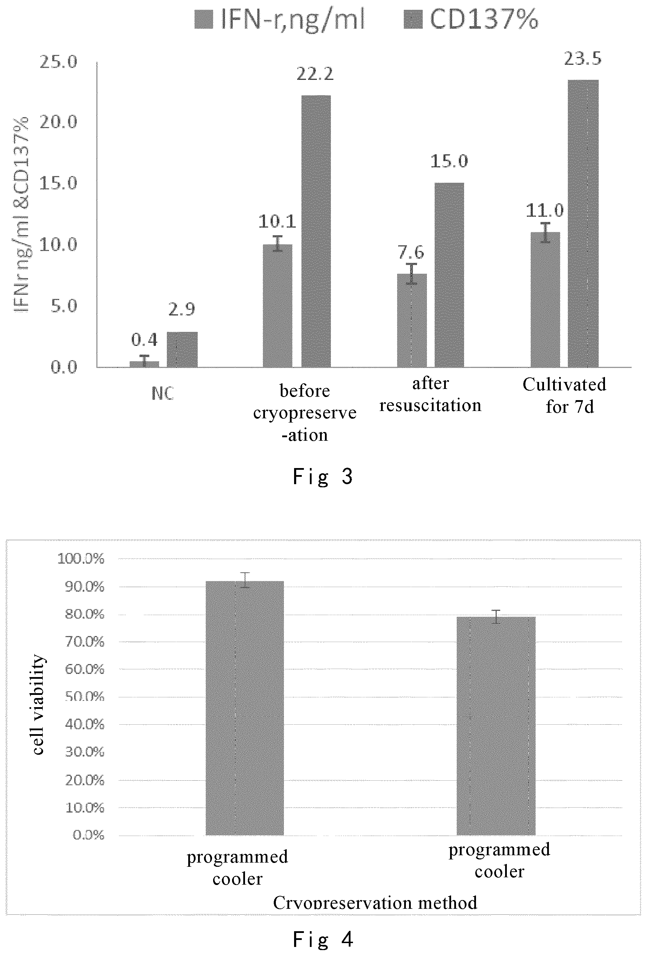

**The tumor antigen response ability of CAR-T cells before and after cryopreservation:** The cryopreserved and resuscitated cells were co-cultured with tumor cells carrying a tumor antigen at 1: 1 overnight, and the culture supernatant was collected to detect the amount of IFNr released by CAR-T cells; Cells were collected and tested for the proportion of CD3 positive cells expressing CD137 to assess tumor antigen response capacity. The data before cryopreservation and the data obtained when the cells were cultured for another 7 days after cryopreservation were used as controls, as shown in Fig. 3. The results showed that the tumor antigen response capacity of the cells did not decrease significantly after the cryopreservation-resuscitation process.

Conclusion: The survival rate of the resuscitated cell preparation after cryopreserved for 3 months was over 90%. The reconstituted frozen CAR-T cell preparation still has proliferation capabilities. There is a reversible decrease in tumor antigen response ability for the resuscitated frozen CAR-T cell preparation, but the ability was restored after continued culture. In summary, the frozen cell preparation maintains the original potency of fresh cells and can be used clinically.

### Example 2 Comparison of cell resuscitation rates under different cryopreservation procedures

After cryopreserved in different cryopreservation procedures, the cells were quickly thawed in 37 °C water and transferred to DMEM pre-warmed at 37 °C, and about 0.3 ml was taken for viability detection. The results showed that, the resuscitation rate of the cells after cryopreserved and resuscitated using the method of the present application was higher than that of the cells resuscitated by cryopreservation and resuscitation method in the control group.

All literatures mentioned in the present application are incorporated herein by reference, as though each one is individually incorporated by reference. Additionally, it should be understood that after reading the above teachings, those skilled in the art can make various changes and modifications to the present invention. These equivalents also fall within the scope defined by the appended claims.

## Claims

1. A cryopreserved (frozen) cell preparation, wherein the preparation comprises:
(1) cells;
(2) A cryopreservation solution comprising: an aqueous solution of sodium chloride, a protective protein, and dimethyl sulfoxide.

2. The preparation of claim 1, wherein the cells are selected from the group consisting of primary T cells, amplificated T cells, gene transducted T cells, stem cells, and combinations thereof.

3. The preparation of claim 1, wherein the cells are selected from the group consisting of suspension-cultured cells and monolayer-cultured cells.

4. The preparation of claim 1, wherein the cell density in the preparation ranges from 1x10⁵-5x10⁸ / ml.

5. The preparation of claim 1, wherein the concentration of sodium chloride in the preparation is from 0.85% to 0.95% (w/v).

6. The preparation of claim 1, wherein the concentration of protective protein in the preparation is from 10% to 90% (w/v).

7. The preparation of claim 1, wherein the concentration of dimethyl sulfoxide in the preparation is from 5% to 40% (w/v).

8. A method for preparing a preparation according to claim 1, wherein the method comprises the steps:
(a) providing cells to be cryopreserved and a cryopreservation diluent, and resuspending the cells with the cryopreservation diluent to obtain a first cryopreservation mixture; wherein the cryopreservation diluent comprises the following components: an aqueous solution of sodium chloride, and a protective protein;
(b) adding the cryopreservation solution to the first cryopreservation mixture, and mixing to obtain a second cryopreservation mixture;
(c) transferring the second cryopreservation mixture to a container and conducting a programmed cooling to below -80 °C to obtain the cryopreserved cell preparation.

9. A resuscitation method for cryopreserved cells, comprising the steps:
(i) providing the cryopreserved cell preparation of claim 1;
(ii) placing the cryopreserved cell preparation to a 35-40 °C constant temperature apparatus for resuscitation to obtain a resuscitated cell-freezing solution complex;
(iii) transferring the cells in the preparation to an injection carrier with a syringe or a disposable connector.

10. A method according to claim 9, wherein the injection carrier is an intravenously injectable solution, preferably selected from the group consisting of sodium chloride injection, multiple electrolytes injection, and amino acid injection.
